(19) Europäisches Patentamt
European Patent Office
Office européen des brevets

(11) Veröffentlichungsnummer : **0 043 949**
**B1**

(12) # EUROPÄISCHE PATENTSCHRIFT

(45) Veröffentlichungstag der Patentschrift : **05.10.83**

(51) Int. Cl.³ : **C 07 C103/19**, C 07 C102/06

(21) Anmeldenummer : **81104876.8**

(22) Anmeldetag : **24.06.81**

(54) **Verfahren zur Herstellung von Cyclopropylcarbonsäureamiden.**

(30) Priorität : **10.07.80 DE 3026094**

(43) Veröffentlichungstag der Anmeldung : **20.01.82 Patentblatt 82/03**

(45) Bekanntmachung des Hinweises auf die Patenterteilung : **05.10.83 Patentblatt 83/40**

(84) Benannte Vertragsstaaten : **BE CH FR GB IT LI NL**

(56) Entgegenhaltungen :
**DE A 1 939 759**
**FR A 2 093 472**

(73) Patentinhaber : **Degussa Aktiengesellschaft**
**Weissfrauenstrasse 9**
**D-6000 Frankfurt am Main 1 (DE)**

(72) Erfinder : **Kleemann, Axel, Dr.**
**Greifenhagenstrasse 9**
**D-6450 Hanau 9 (DE)**
Erfinder : **Schwarze, Werner, Dr.**
**Leerbachstrasse 117**
**D-6000 Frankfurt (Main) (DE)**
Erfinder : **Remmel, Hans**
**Altenmittlauerstrasse 44**
**D-6463 Freigericht 3 (DE)**
Erfinder : **Hohn, Wolfgang**
**Brüder-Grimm-Strasse 23**
**D-6466 Gründau 2 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 043 949 B1

Verfahren zur Herstellung von Cyclopropylcarbonsäureamiden

Die Anmeldung betrifft ein neues Herstellungsverfahren für bestimmte Cyclopropylcarbonsäureamide, bei dem von entsprechenden γ-Chlorbuttersäureestern ausgegangen wird.

Cyclopropylcarbonsäureamide können mit Hilfe von wasserentziehenden Mitteln leicht in die entsprechenden Nitrile überführt werden. Diese dienen ihrerseits als Basis für die Herstellung von verschiedenen cyclopropylgruppenhaltigen Aminen. Durch Hydrierung kann man die Nitrile in die entsprechenden Methylamine überführen. Arbeitet man hierbei in Gegenwart von anderen primären Aminen, so erhält man sekundäre Amine, die beispielsweise Bestandteile von wichtigen Herbiciden sind. So wird z. B. das Cyclopropylmethyl-N-propylamin für die Synthese eines Sojaherbicides verwendet (US-PS 3 546 295).

Cyclopropancarbonsäureamide können beispielsweise durch Umsetzung der Cyclopropancarbonsäureester mit Ammoniak unter Druck bei erhöhten Temperaturen z. B. 80 °C erhalten werden (DE-AS 19 39 759). Bei diesem Verfahren fällt nachteilig ins Gewicht, daß es von Cyclopropanderivaten ausgeht.

Es ist auch bekannt, Cyclopropancarbonsäureester durch Cyclisierung von γ-Chlorbuttersäureestern herzustellen. Hierbei werden letztere z. B. mit t-Natriumamylat in t-Amylalkohol vier Stunden lang zum Sieden erhitzt. Der Cyclopropancarbonsäureäthylester wird hierbei in einer Ausbeute von nur 45 % erhalten (Julia et al, Bull. Soc. Chemie (France) 1960, p. 306 ff). Setzt man γ-Chlorcapronsäureester mit Natriumamid in Äther um, so erhält man, nach einer Reaktionszeit von 4 bis 5 Tagen, den 2-Methylcyclopropylcarbonsäureester in einer Ausbeute von 85 % (Am. Soc. 81, p. 1660-66).

Schließlich ist noch die Umsetzung von γ-Chlorbuttersäureäthylester mit Natriumethylat bekannt. Es werden jedoch nur Ausbeuten von 66 % erhalten (Bunce et al, Organic Preparations and Procedures 6, p. 193-6 (1974)). Auch die Herstellung von Cyclopropancarbonsäureäthylester aus 4-Brombuttersäureester durch Cyclisierung mit NaH, ist bereits beschrieben worden. Man erhält eine Ausbeute von 88 % (I. Chem. Eng. Data *14,* 396 (1969)).

Schließlich ist es auch bekannt, wässrige Cyclopropylcarbonsäureamid-Lösungen herzustellen, indem man in einer ersten Stufe die Umsetzung von 4-Chlorbuttersäuremethylester mit Natriummethylat in einer inerten Gasatmosphäre, in einer nicht reaktiven organischen Flüssigkeit, z. B. in Toluol, unter wasserfreien Bedingungen durchführt. In dieser Stufe betragen die Ausbeuten 92 % der Theorie. In einer zweiten Stufe wird sodann der erhaltene Cyclopropancarbonsäuremethylester mit Natriummethylat in Methanol in Gegenwart von Toluol unter Druck mit Ammoniak zu dem Cyclopropancarbonsäureamid umgesetzt. In dieser Stufe betragen die Ausbeuten 85 bis 90 %. (DE-AS 19 39 759).

Diese vorbekannten Verfahren sind bei ihrer Durchführung sehr aufwendig und kostspielig.

Es wurde nun gefunden, daß man Cyclopropylcarbonsäureamide der allgemeinen Formel (I)

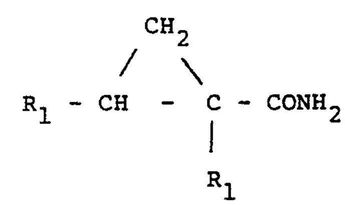

(I)

durch Umsetzung von γ-Chlorbuttersäureester der allgemeinen Formel (II)

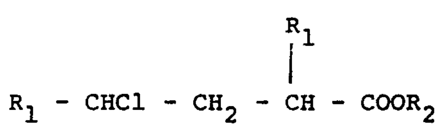

(II)

unter wasserfreien Bedingungen mit Alkalialkoholaten gegebenenfalls kontinuierlich herstellen kann, wenn man die γ-Chlorbuttersäureester der Formel (II) mit Natrium- oder Kaliummethylat in Methanol oder mit Natrium- oder Kaliumäthylat in Äthanol in Gegenwart von flüssigem Ammoniak umsetzt und dabei unter Druckanwendung eine Temperatur einhält, die oberhalb der Siedetemperatur des verwendeten Alkoholes liegt.

In den allgemeinen Formeln (I) und (I') stehen $R_1$ für ein Wasserstoffatom oder für die Methylgruppe und $R_2$ für die Methyl- oder Äthylgruppe.

Es ist durchaus überraschend, daß man unter Einhaltung der genannten Reaktionsbedingungen aus γ-Chlorbuttersäureestern mit den Na/K-Alkoholaten des Methanols und Äthanols allein im Methanol bzw. Äthanol die gewünschten Cyclopropancarbonsäureamide in höchster Ausbeute erhält.

Das erfindungsgemäße Verfahren weist demnach folgende Vorteile auf :

1. Es ist ein Einstufenverfahren zur Herstellung der Cyclopropancarbonsäureamide, während früher nur 2-Stufenverfahren zur Verfügung standen.

2. Verwendung von nur einem Lösungsmittel.

3. Große Einsparung von Zeit und Energie durch sehr viel schnelleren Ablauf der Reaktion.

4. Höhere Ausbeute als beim 2-Stufenverfahren.

5. Die Benutzung des sehr teuren und sensiblen Natriummethylatpulvers, dessen Handhabung gefährlich und dessen Stäube hochtoxisch sind, entfällt. Stattdessen werden die viel ungefährlicheren Natriummethylatlösungen verwendet.

Zweckmäßigerweise wendet man die Alkoholate in mindestens äquimolekularen Mengen an. Vorteilhaft ist es jedoch, einen etwa 10 %-igen Überschuß einzusetzen.

Den wasserfreien Ammoniak verwendet man ebenfalls in mindestens äquimolekularen Menge, jedoch ist hier die Anwendung eines 100 bis 200 %-igen Überschusses vorteilhaft.

Die erfindungsgemäße Reaktion kann bei einer Temperatur zwischen 90 °C und 200 °C durchgeführt werden. Der Temperaturbereich zwischen 140 °C und 160 °C ist jedoch bevorzugt.

Bei der diskontinuierlichen Durchführung des Verfahrens kann beispielsweise so vorgegangen werden, daß man in einem Druckgefäß pro Mol γ-Chlorbüttersäureester 1 bis 1,1 Mol Alkoholat gelöst in Alkohol vorlegt. In den verschlossenen Autoklaven wird dann Ammoniakgas eingepreßt. Dabei ist es vorteilhaft, den obengenannten Überschuß einzuhalten. Der Autoklav wird sodann aufgeheizt. Sobald der Temperaturbereich zwischen 90 °C und 200 °C erreicht ist, wird der Halogenester in den Autoklaven eingepumpt. Dabei läuft die Reaktion in wenigen Minuten ab. Nach Beendigung der Reaktion kann noch kurz nachgeheizt und sodann in an sich bekannter Weise aufgearbeitet werden.

Bei der kontinuierlichen Arbeitsweise wird die oben beschriebene Reaktion in einfacher Weise in einer Druckschlange durchgeführt.

Der Ablauf dieser Reaktion ist durchaus überaschend, da es bekannt ist, daß die Umsetzung von γ-Chlorbuttersäureestern mit Natriumalkoholat nur in geringen Ausbeuten Cyclopropancarbonsäureester liefert und weil es andererseits bekannt ist, daß die γ-Chlorbuttersäureester mit Ammoniak zu Pyrrolidonen reagieren (Siehe Vergleichsbeispiele).

Die für das erfindungsgemäße Verfahren als Ausgangsstoffe benötigten γ-Chlorbuttersäureester können nach an sich bekannten Verfahren aus den entsprechenden Lactonen oder γ-Hydroxi- oder Chlorcarbonsäuren bzw. Chlorcarbonsäurechloriden hergestellt werden (Liebigs Ann. 596, p. 163-164 ; Synthesis 1973, p. 538-539 ; DBP 804 567 ; F.P. 1 080 261 ; US-PS 3 927 074).

## Beispiel 1

Man legt in einem 5 l-Rührautoklaven 594 g Natriummethylat in 1 500 ml Methanol vor, verschließt diesen und drückt nun aus einer Stahlflasche 350 g Ammoniak auf. Nun erhitzt man auf 145 °C. Innerhalb von 2 Stunden pumpt man 1 365 kg γ-Chlorbuttersäuremethylester ein, erhitzt anschließend noch eine Stunde und kühlt ab. Der Autoklav wird entleert, die Mischung mit konz. Schwefelsäure neutralisiert, dann das gebildete Ammonchlorid und Ammonsulfat abgenutscht, mit Methanol gewaschen und das Filtrat aufgearbeitet.

Man erhält 799 g Cyclopropansäureamid, entsprechend 94 % d. Th. Weiße Kristalle, F. 120 °C-121 °C.

## Beispiel 2

Man löst in 1 l absolutem Äthanol 50 g Natrium und gibt die noch warme Lösung in einen 2 l-Autoklaven, verschießt und drückt 80 g Ammoniak auf. Der Autoklav wird hochgeheizt auf 145 °C-150 °C. Nun pumpt man 301 g γ-Chlorcapronsäuremethylester innerhalb einer Stunde ein, erhitzt noch eine Stunde und arbeitet wie in Beispiel 1 beschrieben auf.

Man erhält 180,4 g 2-Methylcyclopropancarbonsäureamid, F. 120 °C, Weiße Kristalle.

Ausbeute 91,1 % d. Th.
Analyse : $C_5H_9ON$ (M = 99)

| | | | |
|---|---|---|---|
| ber. | C 60,6 | H 9,1 | N 14,1 |
| gef. | 60,8 | 9,0 | 13,9 |

## Beispiel 3

Man löst 55,2 g Natrium in 1 Liter Methanol und gibt die Lösung in einen 2 l-Autoklaven, verschließt diesen und drückt 100 g Ammoniak auf. Man erhitzt auf 140 °C und pumpt bei dieser Temperatur innerhalb von 40 Minuten 301 g α-Methyl-γ-chlorbuttersäuremethylester ein, erhitzt anschließend noch eine Stunde und arbeitet wie in Beispiel 1 beschrieben auf.

Man erhält 174,5 g 1-Methylcyclopropancarbonsäureamid, F. 133 °C, weiße Kristalle.

Ausbeute 88,1 % d. Th.
Analyse : $C_5H_9ON$ (M = 99)

| | | | |
|---|---|---|---|
| bar | C 60,6 | H 9,1 | N 14,1 |
| gef. | 60.7 | 8,9 | 14,0 |

Vergleichsbeispiel

a) In einem 2 l-Rührkolben mit Rührwerk legt man 178,2 g Natriummethylat in 570 ml Methanol vor. Innerhalb von 1 Stunde tropft man unter Rückfluß 409,5 g γ-Chlorbuttersäure-methylester ein und erhitzt anschließend noch 2 Stunden unter Rückfluß. Dann wird abgekühlt und 750 ml Methylenchlorid hinzugegeben. Anschließend tropft man 1,5 l Wasser hinzu, schüttelt aus und trennt die $CH_2Cl_2$-Schicht ab. Die Aufarbeitung liefert 198,6 g Cyclopropancarbonsäuremethylester, Kp 114 °C-115 °C, Ausbeute : 66,2 %.

Die destillative Aufarbeitung des Rückstands liefert 81 g γ-Methoxibuttersäuremethylester (20,4 % d. Th.) und 36 g (= 8,8 %) nicht umgesetzten γ-Chlorbuttersäuremethylester, ferner 6,1 g γ-Butyrolacton.

b) Man gibt in einen 2 l-Autoklaven 500 ml Methanol verschließt und drückt 200 ml $NH_3$ aus der Druckflasche auf. Nun erhitzt man auf 150 °C und pumpt innerhalb einer Stunde 273 g γ-Chlorbuttersäuremethylester ein. Anschließend erhitzt man noch 1 Stunde, kühlt dann ab, öffnet den Autoklaven, nutscht das gebildete Ammoniumchlorid (94 g) ab und dampft das Filtrat ein. Es verbleibt ein helles wasserlösliches Öl, Menge : 175,5 g. Vakuumdestillation liefert bei $Kp_{10}$ 128°-129 °C Pyrrolidon-2, Menge : 127,1 g = 74,7 % d. Th.

**Ansprüche**

1. Verfahren zur gegebenenfalls kontinuierlichen Herstellung von Cyclopropylcarbonsäureamiden der allgemeinen Formel (I)

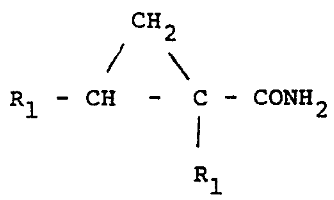

(I)

in der $R_1$ ein Wasserstoffatom oder eine $CH_3$-Gruppe bedeutet, durch Umsetzung von γ-Chlorbuttersäureestern der allgemeinen Formel (II)

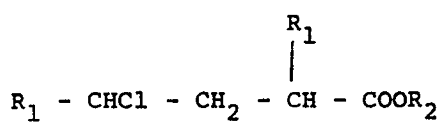

(II)

in der $R_1$ die oben angegebenen Bedeutungen hat und $R_2$ für die Methyl- oder Äthylgruppe steht, mit Alkalialkoholaten unter wasserfreien Bedingungen, dadurch gekennzeichnet, daß die γ-Chlorbuttersäureester der Formel (II) mit Natrium- oder Kaliummethylat in Methanol oder mit Natrium- oder Kaliumäthylat in Äthanol in Gegenwart von Ammoniak umgesetzt werden, wobei unter Druckanwendung eine Temperatur eingehalten wird, die oberhalb der Siedetemperatur des verwendeten Alkohols liegt.

2. Verfahren nach Anspruch 1, dadurch gekennzeichnet, daß man die Alkoholate in mindestens äquimolekularen Mengen einsetzt.

3. Verfahren nach Anspruch 1 oder 2, dadurch gekennzeichnet, daß man die Alkoholate in einem Überschuß von 10 % einsetzt.

4. Verfahren nach den Ansprüchen 1 bis 3, dadurch gekennzeichnet, daß man den wasserfreien Ammoniak in mindestens äquimolekularen Mengen einsetzt.

5. Verfahren nach den Ansprüchen 1 bis 4, dadurch gekennzeichnet, daß man den wasserfreien Ammoniak in einem Überschuß von 100 bis 200 % einsetzt.

6. Verfahren nach den Ansprüchen 1 bis 5, dadurch gekennzeichnet, daß man die Reaktion bei einer Temperatur zwischen 90 °C und 200 °C durchführt.

7. Verfahren nach den Ansprüchen 1 bis 5, dadurch gekennzeichnet, daß man die Reaktion bei einer Temperatur zwischen 140 °C und 160 °C durchführt.

**Claims**

1. A process for the optionally continuous production of cyclopropane carboxamides corresponding to the general formula (I)

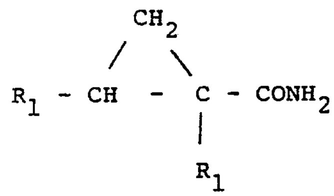

(I)

in which $R_1$ represents a hydrogen atom or a $CH_3$ group, by the reaction of γ-chlorobutyric acid esters corresponding to the general formula (II)

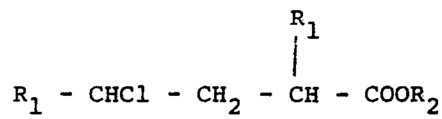

(II)

in which $R_1$ is as defined above and $R_2$ represents the methyl or ethyl group, with alkali alcoholates under anhydrous conditions, characterised in that the γ-chlorobutyric acid esters corresponding to formula (II) are reacted with sodium- or potassium methylate in methanol or with sodium- or potassium ethylate in ethanol in the presence of ammonia, and a temperature is observed while applying a pressure, which temperature is above the boiling temperature of the alcohol which is used.

2. A process according to claim 1, characterised in that the alcoholates are used in at least equimolecular quantities.

3. A process according to claim 1 or 2, characterised in that the alcoholates are used in an excess of 10 %.

4. A process according to claims 1 to 3, characterised in that the anhydrous ammonia is used in at least equimolecular quantities.

5. A process according to claims 1 to 4, characterised in that the anhydrous ammonia is used in an excess of from 100 to 200 %.

6. A process according to claims 1 to 5, characterised in that the reaction is carried out at a temperature of from 90 to 200 °C.

7. A process according to claims 1 to 5, characterised in that the reaction is carried out at a temperature of from 140 to 160 °C.

**Revendications**

1. Procédé pour la fabrication, éventuellement en continu, de cyclopropanecarboxamides répondant à la formule générale (I)

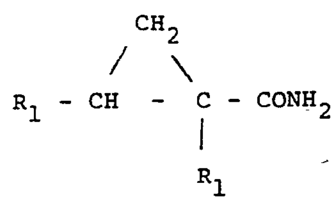

(I)

où $R_1$ est un atome d'hydrogène ou un groupe $CH_3$, par réaction d'esters γ-chlorobutyriques de formule générale (II)

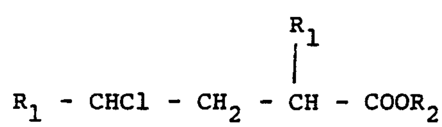

(II)

où $R_1$ a la signification indiquée ci-dessus et $R_2$ représente un groupe méthyle ou éthyle, avec des alcoolates alcalins, dans des conditions anhydres, procédé caractérisé en ce que l'on fait réagir l'ester γ-chlorobutyrique de formule (II) avec du méthylate de sodium ou de potassium dans le méthanol, ou de l'éthylate de sodium ou de potassium dans l'éthanol, en présence d'ammoniac, pendant que, en utilisant une pression, on maintient une température qui se situe au-dessus de la température d'ébullition de l'alcool utilisé.

2. Procédé suivant la revendication 1, caractérisé en ce que l'on utilise les alcoolates en proportions qui sont au moins équimoléculaires.

3. Procédé suivant l'une quelconque des revendications 1 et 2, caractérisé en ce que l'on utilise les alcoolates en excès d'environ 10 %.

4. Procédé suivant l'une quelconque des revendications 1 à 3, caractérisé en ce que l'on utilise l'ammoniac anhydre en proportion au moins équimoléculaire.

5. Procédé suivant l'une quelconque des revendications 1 à 4, caractérisé en ce que l'on utilise l'ammoniac anhydre en excès de 100 à 200 %.

6. Procédé suivant l'une quelconque des revendications 1 à 5, caractérisé en ce que l'on effectue la réaction à une température qui se situe entre 90 et 200 °C.

7. Procédé suivant l'une quelconque des revendications 1 à 5, caractérisé en ce que l'on effectue la réaction à une température entre 140 °C et 160 °C.